(19) **Europäisches Patentamt**

**European Patent Office**

**Office européen des brevets**

(11) Veröffentlichungsnummer: **0 304 627 B1**

(12) **EUROPÄISCHE PATENTSCHRIFT**

(45) Veröffentlichungstag der Patentschrift: **24.02.93**

(51) Int. Cl.⁵: **A61K 7/22**

(21) Anmeldenummer: **88111765.9**

(22) Anmeldetag: **21.07.88**

(54) **Antimikrobiell wirksame, aromatisierte Zubereitungen.**

(30) Priorität: **30.07.87 DE 3725248**

(43) Veröffentlichungstag der Anmeldung:
**01.03.89 Patentblatt 89/09**

(45) Bekanntmachung des Hinweises auf die
Patenterteilung:
**24.02.93 Patentblatt 93/08**

(84) Benannte Vertragsstaaten:
**AT BE CH DE ES FR GB GR IT LI LU NL SE**

(56) Entgegenhaltungen:
**EP-A- 0 026 252**
**EP-A- 0 110 568**
**EP-A- 0 185 971**
**FR-A- 2 115 375**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Wülknitz, Peter, Dr.
Hospitalstrasse 18
W-4000 Düsseldorf-Benrath(DE)**
Erfinder: **Lehmann, Rudolf, Dr.
Schnugsheide 2
W-5653 Leichlingen(DE)**
Erfinder: **Plöger, Walter, Dr.
Holbeinweg 11
W-4010 Hilden(DE)**
Erfinder: **Hill, Karlheinz, Dr.
Am Hasenbusch 1
W-4006 Erkrath(DE)**
Erfinder: **Förg, Franz, Dr.
Rotdornweg 10
W-4018 Langenfeld(DE)**

**Beschreibung**

Die Erfindung betrifft wässrige Zubereitungen mit einem Gehalt an antimikrobiell wirksamen Biguanidverbindungen, deren Wirkung verstärkenden Alkylglycosid-Tensiden und homogen solubilisierten, wasserunlöslichen Aromaölen, die sich bevorzugt als antiseptische Mundwässer eignen.

Als Mundwässer werden üblicherweise wässrig-alkoholische Zubereitungen von Aromaölen verwendet, die bevorzugt antimikrobielle Verbindungen und oberflächenaktive Stoffe enthalten. Die Produkte sind entweder als klare oder getrübte, gebrauchsfertige Lösungen im Handel, die unverdünnt angewandt werden, oder bevorzugt in Form von Konzentraten, die vor der Anwendung mit Wasser auf Gebrauchskonzentration gebracht werden.

Es besteht dabei das Ziel, mit möglichst geringen Konzentrationen an antibakteriellen Verbindungen eine ausreichende und möglichst gegen die grampositiven Keime spezifische Wirkung zu erreichen, da diese bei der Entstehung des Zahnbelags und damit für die Kariesentwicklung eine besondere Rolle spielen.

Aus der EP-A-185 971 ist bekannt, daß dies durch eine Kombination von antibakteriell wirksamen Biguanidverbindungen mit Alkylglycosid-Tensiden erreicht werden kann.

Mundwässer sollen aber organoleptisch akzeptable Aromaöle enthalten, die einerseits einen Beitrag zu der antimikrobiellen Wirkung leisten, andererseits dem Mundwasser einen angenehmen und erfrischenden Geschmack und bei der Anwendung eine desodorisierende Wirkung verleihen. Die dafür geeigneten wasserunlöslichen Aromaöle müssen in dem Mundwasser bzw. in dem Mundwasserkonzentrat stabil und homogen solubilisiert werden, damit es nicht zu einer vorzeitigen Entmischung des Konzentrats oder der daraus hergestellten Gebrauchslösung kommt. Hierfür werden üblicherweise niedere Alkohole, insbesondere Ethanol, und oberflächenaktive Stoffe eingesetzt. Man ist jedoch bestrebt, den Gehalt an Ethanol sowohl aus Kostengründen als auch wegen der durch höhere Ethanolkonzentrationen verursachten Schleimhautreizungen möglichst nierdrig zu halten. Dies bedingt einen erhöhten Einsatz besonders wirksamer Aromaöl-Solubilisatoren. Es wurde aber beobachtet, daß viele bekannte Solubilisatoren für Aromaöl die potenzierte antimikrobielle Wirkung des Systems aus antimikrobiell wirksamen Biguanidverbindungen und Alkylglycosid-Tensiden wieder verringern, so daß eine Erhöhung der Konzentration an antimikrobiellen Verbindungen oder der Zusatz stärker wirksamer bakterizider Substanzen, z. B. von Wasserstoffperoxid oder von höheren Ethanolkonzentrationen erforderlich wäre, um eine ausreichende antibakterielle Wirkung zu erhalten. Solche Maßnahmen sind aber bei Zubereitungen, die auf Schleimhäute angewendet werden, äußerst unerwünscht, insbesondere, wenn sich die Anwendungsdauer über einen längeren Zeitraum erstreckt.

Es bestand daher die Aufgabe, antimikrobiell wirksame, aromatisierte Zubereitungen zu finden, die eine niedrige Konzentration an antimikrobiell wirksamen Biguanidverbindungen und homogen solubilisierte Aromaöle und bei Anwendungskonzentration nicht mehr als maximal 15 Gew.- % Ethanol enthalten. Es wurde gefunden, daß diese Aufgabe unter Verwendung spezieller Lösungsvermittler und durch eine nach Art und Menge spezifizierte Auswahl von Komponenten in besonders wirksamer Weise gelöst werden kann.

Gegenstand der Erfindung sind wässrige, homogene Zubereitungen mit einem Gehalt antimikrobiell wirksamer Biguanidverbindungen und Alkylglycoside, gekennzeichnet durch einen Gehalt von

(A) 0,0025-0,1 Gew.- % einer antimikrobiell wirksamen Biguanidverbindung,

(B) 0,005-0,2 Gew.- % eines Alkylglycosids mit 8-16 C-Atomen in der Alkylgruppe und einem mittleren Oligomerisierungsgrad des Glycosidrests von 1-8,

(C) 0,01-0,3 Gew.-% eines wasserunlöslichen Aromaöls,

(D) 0,01-0,3 Gew.- % eines Lösungsvermittlers aus der Gruppe der oxethylierten Fettsäureglyceride, der oxethylierten Fettsäuresorbitanpartialester oder der Fettsäurepartialester von Glycerin- oder Sorbitan-Oxethylaten.

Der erfindungsgemäßen Zubereitungen sind frei von anderen antimikrobiellen Zusätzen wie z. B. Wasserstoffperoxid oder anderen Peroxyverbindungen. Sie sind ganz oder weitgehend frei von niederen Alkoholen, können aber Ethanol in einer Menge von 0-15 Gew.- % enthalten.

Die antimikrobiell wirksamen Biguanidverbindungen sind bevorzugt in sehr niedriger Konzentration von 0,0025-0,04 Gew.- % enthalten. Als antimikrobiell wirksame Biguanidverbindung wird bevorzugt das aus GB-A-705 838 bekannte 1,1'-Hexamethylen-bis-(4-chlorphenyl)-biguanid ("Chlorhexidin") in Form eines wasserlöslichen, physiologisch verträglichen Salzes, z. B. in Form des Acetats oder als Gluconat eingesetzt. Andere erfindungsgemäß geeignete antimikrobielle Biguanidverbindungen sind z. B. die aus Polyhexamethylenbiguanidverbindungen des Typs Vantocil[(R)] IB (ICI), das aus DE-A-1 964 196 bekannte 1,6-Bis-(4-chlorbenzylbiguanido) -hexan (Fluorhexidin) ferner, die aus US-A-2 684 924, US-A-2 990 425, US-A-3 468 898, US-A-4 022 834, US-A-4 053 636 und US-A-4 198 392 bekannten antimikrobiellen Biguanid-

2

Verbindungen.

Alkylglycoside, ihre Herstellung und ihre Verwendung als oberflächenaktive Stoffe sind beispielsweise aus US-A-3 839 318, US-A-3 707 535, US-A-3 547 828, DE-A-1 943 689, DE-A-2 036 472 und DE-A-3 001 064 sowie EP-A-77167 bekannt. Ihre Herstellung erfolgt insbesondere durch Umsetzung von Glucose oder Oligosacchariden mit primären Alkoholen mit 8-16 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise 8 geeignet sind. Bevorzugt geeignete Alkylglycoside für die Herstellung der erfindungsgemäßen Zubereitungen sind solche mit 8-14 C-Atomen und mindestens einer Verzweigung in der Alkylgruppe und einem mittleren Oligomerisierungsgrad des Glycosidrestes von 1-4. Der Oligomerisierungsgrad ist dabei ein statistischer Mittelwert, dem eine für solche technischen Produkte übliche Homologenverteilung zugrunde liegt.

Ein besonders bevorzugtes Alkylglycosid ist das Isotridecylglucosid mit einem Oligomerisierungsgrad (OG) von ca. 3.

Als wasserunlösliche Aromaöle kommen alle für Mund- und Zahnpflegemittel gebräuchlichen natürlichen und synthetischen Aromen infrage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Lösungsvermittler aus der Gruppe der oxethylierten Fettsäureglyceride umfassen vor allem Anlagerungsprodukte von 20-60 Mol Ethylenoxid an Mono- und Diglyceride von linearen Fettsäuren mit 12-18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20-60 Mol Ethylenoxid an Sorbitanmonoester und Sorbitandiester von Fettsäuren mit 12-18 C-Atomen. Ebenfalls geeignete Lösungsvermittler sind Fettsäurepartialester von Glycerin- oder Sorbitan-Oxethylaten; das sind bevorzugt Mono- und Diester von $C_{12}$-$C_{18}$-Fettsäuren und Anlagerungsprodukten von 20-60 Mol Ethylenoxid an 1 Mol Glycerin oder an 1 Mol Sorbit.

Die erfindungsgemäßen Zubereitungen enthalten bevorzugt als Lösungsvermittler Anlagerungsprodukte von 20-60 Mol Ethylenoxid an gehärtetes oder ungehärtetes Ricinusöl (d. h. an Oxystearinsäure- oder Ricinolsäure-triglycerid), an Glycerin-mono- und/oder distearat oder an Sorbitanmono- und/oder distearat.

Neben den genannten obligatorischen Komponenten können die erfindungsgemäßen Zubereitungen noch weitere, an sich bekannte und in antimikrobiellen Zubereitungen für den Bereich der Körperhygiene übliche Komponenten zur Verbesserung des Aussehens, des Geschmacks oder der Handhabung enthalten. Zur Verbesserung des Aussehens können z. B. physiologisch unbedenkliche Farbstoffe, Trübungs- oder Perlglanzmittel zugegeben werden. Zur Verbesserung des Geschmackes können natürliche oder synthetische, bevorzugt von kariogenen Kohlehydraten freie Süssungsmittel zugegeben werden. Hierzu gehören z.B. Saccharin-Natrium, Cyclamate, Acesulfam-Kalium, Aspartame[R] (L-Aspartyl-L-phenylalaninmethylester), Glycerin, Sorbit, Mannit oder Xylit.

Gegenstand der Erfindung sind auch Konzentrate von fester oder flüssiger Konsistenz, welche die obligatorischen Komponenten A, B, C, und D in einer solchen Konzentration und in einem solchen Konzentrationsverhältnis zueinander enthalten, daß sich durch Auflösen des Konzentrats in Wasser oder durch Verdünnung mit Wasser in einem Gewichtsverhältnis von 1:1 bis 1:500 erfindungsgemäße Zubereitungen ergeben.

Sind solche Konzentrate z. B. flüssig, so können sie auch Anteile von mehr als 15 Gew.-% Ethanol enthalten, wenn bei der zur Herstellung der Anwendungskonzentration vorgesehenen Verdünnung mit Wasser die Konzentration an Ethanol auf 15 Gew.-% oder darunter sinkt.

Sollen solche Konzentrate z. B. in Form von Pulvern, Granulaten oder Tabletten vorliegen, so enthalten sie neben den obligatorischen Komponenten A, B, C und D noch feste oder pulverförmigen Füllstoffe und Hilfsmittel, welche dem Gemisch entweder eine freifließende pulverförmige Struktur verleihen oder die Herstellung fließfähiger Granulate oder bruchfester Tabletten ermöglichen.

Solche pulverförmigen Füllstoffe und Hilfsmittel können zum Beispiel sein: pulverförmiger Sorbit, Mannit oder Xylit, wasserlösliche Stärke, pulverförmige Kieselsäuregele (z. B. Aerosil), Salze wie z. B. Natriumchlorid, Natriumbicarbonat oder Magnesiumsulfat. Zur Herstellung von Sprudeltabletten, die sich besonders rasch und unter Kohlendioxid-Entwicklung in Wasser auflösen, können Kombinationen aus Natriumbicarbonat und pulverförmigen
organischen Säuren, z. B. Citronensäure, Weinsäure oder Äpfelsäure enthalten sein. Die genannten pulverförmigen Füllstoffe und Hilfsmittel sind bevorzugt in einer Menge von 80-98 Gew.- % des gesamten, festen Konzentrats enthalten.

Die Herstellung der erfindungsgemäßen Zubereitungen und der flüssigen Konzentrate erfolgt durch einfaches Auflösen der Komponenten in Wasser. Die Herstellung von festen Granulaten, Tabletten oder Sprudeltabletten kann nach der für solche Produkte üblichen Misch-, Granulier- oder Tablettiertechnik erfolgen.

Die folgenden Beispiele sollen den Erfindungsgegenstand näher erläutern ohne ihn hierauf zu beschränken.

**Beispiele**

## 1. Mundwasser (Anwendungskonzentration)

|  | 1.1 | 1.2 | 1.3 |
|---|---|---|---|
| Ethanol (99%ig, unvergällt) | 5,0 | 5,0 | 10,0 |
| Chlorhexidin-digluconat | 0,03 | 0,015 | 0,015 |
| Isotridecyl-glucosid (OG=3) | 0,1 | 0,1 | - |
| n-$C_{12}$/$C_{14}$-alkyl-glucosid (OG:2,2) | - | - | 0,2 |
| n-$C_8$/$C_{10}$-alkyl-glucosid (OG:1,8) | - | - | 0,1 |
| HR 60[1] | 0,1 | 0,1 | - |
| GMS 20[2] | - | - | 0,2 |
| Aromaöl[3] | 0,1 | 0,1 | 0,2 |
| Farbstoff blau[4] (1%ig in $H_2O$) | 0,1 | 0,1 | 0,1 |
| Sorbit (70%ig) | 3,0 | 3,0 | 3,0 |
| Saccharin-Natrium | 0,05 | 0,05 | 0,05 |
| Wasser | ad 100 g | ad 100 g | ad 100 g |

## 2. Mundwasser-Konzentrate

### 2.1 Flüssige Konzentrate

|  | 2.1 20fach-Konzentrat | 2.2 50fach-Konzentrat |
|---|---|---|
| Ethanol (99%ig, unvergällt) | 40,0 g | 40,0 g |
| Isotridecyl-glucosid (OG:3) | 2,0 g | 5,0 g |
| HR 60[1] | 2,0 g | 5,0 g |
| Aromaöl[3] | 2,0 g | 5,0 g |
| Farbstoff blau[4] (1%ig in $H_2O$) | 1,0 g | 2,5 g |
| Chlorhexidin-digluconat 20%ig in $H_2O$ | 1,5 g | 3,75 g |
| Saccharin-Natrium | 1,0 g | 2,5 g |
| Wasser | 50,5 g | 36,25 g |
|  | 100,0 g | 100,0 g |

### 2.3 Pulverförmiges, tablettierbares Mundwasserkonzentrat (zur Auflösung in Wasser im Gewichtsverhältnis 1:100)

| | |
|---|---|
| Sorbit (pulverförmig) | 50,0 g |
| $NaHCO_3$ | 19,0 g |
| Stärke (wasserlöslich) | 1,0 g |
| Chlorhexidin-acetat | 3,0 g |
| Isotridecyl-glucosid (OG:3) | 5,0 g |
| HR 60[1] | 1,0 g |
| Aromaöl[3] | 1,0 g |
| Citronensäure | 20,0 g |

1)  Anlagerungsprodukt von 60 Mol Ethylenoxid an 1 Mol hydriertes Ricinusöl

2)  Anlagerungsprodukt von 20 Mol Ethylenoxid an Glycerinmonostearat

3)  Pfefferminzöl

4)  L-Blau 4 (Acid Blue 9, disodium salt, FD & C Blue No. 1, C.I. No. 42090)

3. Mikrobizide Wirksamkeit

Die mikrobizide Wirksamkeit der erfindungsgemäßen Kombinationen und entsprechender Zusammensetzungen, bei welchen einzelne der obligatorischen Komponenten fehlen, wurde gegenüber folgenden Testkeimsuspensionen bestimmt:

A) Staphylococcus aureus $2 \times 10^9$ Keime/ml

B) Streptococcus mutans $1 \times 10^9$ Keime/ml

Die Abtötungszeiten der zu untersuchenden Produkte wurden mit Hilfe des Suspensionstestes ermittelt. Unter Verwendung von Wasser einer Härte von 17° dH wurden Testlösungen gemäß Tabelle I hergestellt, von welchen die Lösungen 3.1 und 3.8 die erfindungsgemäße Zusammensetzung aufwiesen.

Bei Raumtemperatur wurden jeweils 0,1 ml Testkeimsuspension in Reagenzgläser pipettiert und mit jeweils 10 ml der oben beschriebenen Testlösungen vermischt. Nach unterschiedlichen Einwirkungszeiten bis zu 15 Minuten wurden den Reagenzgläsern mit Hilfe einer Impföse zweimal ca. 0,05 ml Material entnommen und Substratkulturen sowohl in flüssigen als auch auf festem Nährmedium (Agar) angelegt. Diese enthielten als Enthemmer 3 % Tween 80, 0,3 % Lecithin und 0,1 % Histidin. Das Nährmedium bestand aus 3,0 gewichtsprozentiger Casein-Soja-Bouillon (Merck), die im Falle des festen Nährmediums jeweils 1,2 Gew.-% Agar enthielt. Die Proben wurden bei 37 ° bebrütet. Nach frühestens 2 Tagen wurden die Kulturen makroskopisch auf Wachstum beurteilt und auf diesem Weg die Abtötungszeit oder der Restkeimgehalt ermittelt.

In der nachfolgenden Tabelle I bedeuten "-" keine, "+" weniger als 50, "+ +" weniger als 200 und "+ + +" mehr als 200 Restkeime nach der in Klammern angegebenen Einwirkungszeit.

## Tabelle I

| | 3.1 | 3.2 | 3.3 | 3.4 | 3.5 | 3.6 | 3.7 | 3.8 |
|---|---|---|---|---|---|---|---|---|
| Chlorhexidin-digluconat | 0,015 | 0,015 | 0,015 | 0,015 | – | 0,015 | – | 0,015 |
| Isotridecyl-glucosid (O.G.:3,0) | 0,1 | 0,1 | 0,1 | 0,1 | 0,1 | – | – | 0,1 |
| Pfefferminzöl | 0,1 | 0,1 | – | – | 0,1 | 0,1 | 0,1 | 0,1 |
| HR 60 | 0,1 | – | 0,1 | – | 0,1 | 0,1 | 0,1 | 0,1 |
| Ethanol (99%ig, unvergällt) | 5,0 | 5,0 | 5,0 | 5,0 | 5,0 | 5,0 | 5,0 | 10,0 |
| Sorbit (70%ig) | 3,0 | 3,0 | 3,0 | 3,0 | 3,0 | 3,0 | 3,0 | 3,0 |
| Saccharin-Natrium | 0,05 | 0,05 | 0,05 | 0,05 | 0,05 | 0,05 | 0,05 | 0,05 |
| Wasser | ad 100 | ad 100 | ad 100 | ad 100 | ad 100 | ad 100 | ad 100 | ad 100 |
| Bemerkungen | klar | trübe | bitter | bitter | klar | klar | klar | klar |
| Abtötungszeiten (Minuten, Subkultur flüssig) | | | | | | | | |
| Staphylococcus aureus | 5 | 1 | 15 | 1 | 15 | 15 | 15 | 1 |
| Streptococcus mutans | 2 | 5 | 15 | 1 | 15 | 15 | 15 | 5 |
| Restkeimgehalt (Einwirkzeit, Subkultur fest (Agarnährboden)) | | | | | | | | |
| Staphylococcus aureus | -(1) | +(1) | +(2) | -(1) | ++(15) | +++(2) | +++(15) | -(1) |
| Streptococcus mutans | -(1) | -(1) | ++(1) | -(1) | +(15) | ++(2) | ++(15) | -(1) |

Aus den Ergebnissen gemäß Tabelle I ist ersichtlich, daß nur die erfindungsgemäßen Zusammensetzungen 3.1 und 3.8 alle Anforderungen bezüglich Klarheit, Geschmack und ausreichender antibakterieller Wirkung aufweisen.

8

**Patentansprüche**

1. Wässrige, homogene Zubereitungen mit einem Gehalt antimikrobiell wirksamer Biguanidverbindungen und Alkylglycoside, gekennzeichnet durch einen Gehalt von

     (A) 0,0025-0,1 Gew.- % einer antimikrobiell wirksamen Biguanidverbindung,

     (B) 0,005-0,2 Gew.- % eines Alkylglycosids mit 8-16 C-Atomen in der Alkylgruppe und einen mittleren Oligomerisierungsgrad des Glycosidrestes von 1-8

     (C) 0,01-0,3 Gew.- % eines wasserunlöslichen Aromaöls

     (D) 0,01-0,3 Gew.- % eines Lösungsvermittlers aus der Gruppe der oxethylierten Fettsäureglyceride, der oxethylierten Fettsäuresorbitanpartialester oder der Fettsäurepartialester von Glycerin- oder Sorbitan- Oxethylaten.

2. Zubereitungen nach Anspruch 1, dadurch gekennzeichnet, daß zusätzlich 0-15 Gew.- % Ethanol enthalten sind.

3. Zubereitungen nach Anspruch 1 oder 2, dadurch gekennzeichnet, daß die antimikrobiell wirksame Biguanidverbindung in einer Menge von 0,0025-0,04 Gew.- % enthalten ist.

4. Zubereitungen nach einem der Ansprüche 1-3, dadurch gekennzeichnet, daß als antimikrobiell wirksame Biguanidverbindungen 1,1´ -Hexamethylen- bis- (4-chlorphenyl)- biguanid (Chlorhexidin) in Form eines wasserlöslichen, physiologisch verträglichen Salzes enthalten ist.

5. Zubereitungen nach einem der Ansprüche 1-4, dadurch gekennzeichnet, daß als Alkylglycosid ein solches mit 8-14 C-Atomen und mindestens einer Verzweigung in der Alkylgruppe und einem mittleren Oligomerisierungsgrad des Glycosidrestes von 1-4 enthalten ist.

6. Zubereitungen nach einem der Ansprüche 1-5, dadurch gekennzeichnet, daß als wasserunlösliches Aromaöl wenigstens eines aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl oder Citrusöl oder eine oder mehrere isolierte oder synthetisch erzeugte Komponenten dieser Öle enthalten sind.

7. Zubereitungen nach einem der Ansprüche 1-6, dadurch gekennzeichnet, daß als Lösungsvermittler Anlagerungsprodukte von 20-60 Mol Ethylenoxid an gehärtetes oder ungehärtetes Ricinusöl enthalten sind.

8. Feste oder flüssige Konzentrate zur Herstellung von Zubereitungen nach einem der Ansprüche 1-7, dadurch gekennzeichnet, daß die Komponenten A, B, C und D in einer solchen Konzentration enthalten sind, daß sich durch Auflösen in oder Verdünnung mit Wasser im Gewichtsverhältnis 1:1 bis 1:500 Zubereitungen gemäß einem der Ansprüche 1-8 ergeben.

**Claims**

1. Water-based, homogeneous preparations containing antimicrobial biguanide compounds and alkyl glycosides, characterized by a content of

     (A) 0.0025 to 0.1% by weight of an antimicrobial biguanide compound.

     (B) 0.005 to 0.2% by weight of an alkyl glycoside containing 8 to 16 C atoms in the alkyl group and having an average degree of oligomerization of the glycoside component of 1 to 8,

     (C) 0.01 to 0.3% by weight of a water-insoluble aromatic oil,

     (D) 0.01 to 0.3% by weight of a solubilizer from the group consisting of ethoxylated fatty acid glycerides, ethoxylated fatty acid sorbitan partial esters or fatty acid partial esters of glycerol or sorbitan ethoxylates.

2. Preparations as claimed in claim 1, characterized in that they additionally contain 0 to 15% by weight ethanol.

3. Preparations as claimed in claim 1 or 2, characterized in that the antimicrobial biguanide compound is present in a quantity of 0.0025 to 0.04% by weight.

**4.** Preparations as claimed in any of claims 1 to 3, characterized in that they contain 1,1'-hexamethylene-bis-(4-chlorophenyl)-biguanide (chlorhexidine) in the form of a water-soluble, physiologically compatible salt as the antimicrobial biguanide compound.

**5.** Preparations as claimed in any of claims 1 to 4, characterized in that the alkyl glycoside is one containing 8 to 14 carbon atoms and having at least one branch in the alkyl group and an average degree of oligomerization of the glycoside component of 1 to 4.

**6.** Preparations as claimed in any of claims 1 to 5, characterized in that the water-insoluble aromatic oil is at least one selected from the group consisting of peppermint oil, spearmint oil, anise oil, star anise oil, caraway oil, eucalyptus oil, fennel oil, cinnamon oil, clove oil, geranium oil, sage oil, pimento oil, thyme oil, marjoram oil, basil oil or citrus oil or one or more isolated or synthetic components of these oils.

**7.** Preparations as claimed in any of claims 1 to 6, characterized in that they contain adducts of 20 to 60 mol ethylene oxide with hydrogenated or non-hydrogenated castor oil as solubilizers.

**8.** Solid or liquid concentrates for making the preparations claimed in any of claims 1 to 7, characterized in that components A, B, C and D are present in such a concentration that preparations of the type claimed in any of claims 1 to 8 are obtained by dissolution in or dilution with water in a ratio by weight of 1:1 to 1:500.

**Revendications**

**1.** Préparations aqueuses et homogènes renfermant des composés biguanides à activité antimicrobienne et des alkylglucosides, caractérisées par une concentration de
(A) 0,0025 à 0,1 % en poids d'un composé biguanide à activité antimicrobienne,
(B) 0,005 à 0,2 % en poids d'un alkylglucoside avec 8 à 16 atomes de C dans le groupe alkyle et un degré d'oligomérisation moyen du radical glucoside compris entre 1 et 8,
(C) 0,01 à 0,3 % en poids d'une huile aromatique insoluble dans l'eau,
(D) 0,01 à 0,3 % en poids d'un solubiliseur appartenant au groupe constitué des glycérides d'acides gras éthoxylés, des esters partiels de sorbitane d'acides gras éthoxylés ou des esters partiels d'acides gras d'éthoxylates de glycérine ou de sorbitane.

**2.** Préparations selon la revendication 1, caractérisées en ce qu'elles renferment en plus 0 à 15 % en poids d'éthanol.

**3.** Préparations selon la revendication 1 ou 2, caractérisées en ce que le composé biguanide à activité antimicrobienne est contenu dans une proportion allant de 0,0025 à 0,04 % en poids.

**4.** Préparations selon l'une des revendications 1 à 3, caractérisées en ce qu'elles contiennent comme composés biguanides à activité antimicrobienne du 1,1'-hexaméthylène-bis-(4-chlorophényl)-biguanide (chlorhexidine), sous la forme d'un sel soluble dans l'eau, physiologiquement compatible.

**5.** Préparations selon l'une des revendications 1 à 4, caractérisées en ce que l'alkylglucoside qu'elle contiennent comporte 8 à 14 atomes de C et au moins une ramification dans le groupe alkyle, tout en présentant un degré d'oligomérisation moyen du radical glucoside compris entre 1 et 4.

**6.** Préparations selon l'une des revendications 1 à 5, caractérisées en ce que l'huile aromatique soluble dans l'eau qu'elles contiennent est au moins une huile faisant partie du groupe des essences/huiles de menthe poivrée, menthe crépue, anis, anis étoilé, carvi, eucalyptus, fenouil, cannelle, oeillette, géranium, sauge, piment, thym, marjolaine, basilic, ou citrus, ou un ou plusieurs composants isolés de ces essences/huiles ou d'origine synthétique.

**7.** Préparations selon l'une des revendications 1 à 6, caractérisées en ce qu'elles contiennent comme solubiliseur des produits d'addition de 20 à 60 moles d'oxyde d'éthylène à de l'huile de ricin durcie ou non durcie.

8. Concentrés solides ou liquides pour la confection des préparations selon l'une des revendications 1 à 7, caractérisés en ce que les composants A, B, C et D sont contenus dans une concentration telle qu'ils donnent, par dissolution dans ou par dilution à l'eau dans un rapport pondéral compris entre 1:1 et 1:500, des préparations selon l'une des revendications 1 à 7.